# EUROPEAN PATENT SPECIFICATION

(11) **EP 2 311 472 B1**
(45) Date of publication and mention of the grant of the patent: **05.03.2014**
(21) Application number: 09766645.7
(22) Date of filing: 16.06.2009
(51) Int. Cl.: A61K 35/64, A61K 31/353, A61P 25/28, A61P 43/00

(54) **COGNITIVE DISORDER-AMELIORATING AGENT**
MITTEL ZUR LINDERUNG VON KOGNITIVEN STÖRUNGEN
AGENT POUR L' AMÉLIORATION DES TROUBLES COGNITIFS

(30) Priority: 17.06.2008 JP 2008158085
(43) Date of publication of application: 20.04.2011
(73) Proprietor: Japan Royal Jelly Co., Ltd., Chuo-ku Tokyo 104-0031 (JP); Tohoku University, Aoba-ku Sendai-shi Miyagi 980-8577 (JP)
(72) Inventor: YAMAGUCHI, Kikuji, Shizuoka 413-0016 (JP); OHIZUMI, Yasushi, Miyagi 980-8577 (JP); YAMAKUNI, Tohru, Miyagi 980-8577 (JP); NAKAJIMA, Akira, Miyagi 980-8577 (JP); IWABUCHI, Yoshiharu, Miyagi 980-8577 (JP); SHIBUYA, Masatoshi, Miyagi 980-8577 (JP)
(74) Representative: HOFFMANN EITLE
(86) International application number: PCT/JP2009/060937
(87) International publication number: WO 2009/154197

(56) References cited:
- WO-A1-2005/082351
- JP-A- 2001 157 556
- JP-A- 2002 060 340
- JP-A- 2003 026 533
- JP-A- 2004 075 543
- JP-A- 2004 175 695
- JP-A- 2004 203 784
- JP-A- 2005 060 595
- JP-A- 2006 321 732
- JP-A- 2006 327 970
- DATABASE WPI Week 200062 Thomson Scientific, London, GB; AN 2000-638916 XP002666087, & CN 1 143 501 A (QIAN T) 26 February 1997 (1997-02-26)
- '[Ryoyaku Kuchi ni Umashi] Rojinsei Chihosho Seiriteki Roka to Byoteki Roka' SANKEI SHIMBUN 16 February 1999, TOKYO CHOKAN, page 17, XP008143126
- BUNTETSU HAN ET AL.: 'Kokeiten (Rhodiola sacra S.H.Hu no Chikabu) no Prolyl Endopeptidase Sogai Kassei Seibun ni Kansuru Kenkyu' JOURNAL OF TRADITIONAL MEDICINES vol. 15, no. 5, 27 February 1999, pages 354 - 355, XP008141050
- YAN, J.J. ET AL.: 'Protection against beta-amyloid peptide toxicity in vivo with long-term administration of ferulic acid' BR J PHARMACOL vol. 133, no. 1, 2001, pages 89 - 96, XP008140992
- HIROKO ISHII: 'Ferulic Acid no Kinosei' NEW FOOD INDUSTRY vol. 48, no. 12, 01 December 2006, pages 1 - 3, XP008141020
- LI, S. ET AL.: 'Isolation and syntheses of polymethoxyflavones and hydroxylated polymethoxyflavones as inhibitors of HL-60 cell lines' BIOORG MED CHEM vol. 15, no. 10, 2007, pages 3381 - 3389, XP022024195
- SMITH, J.A. ET AL.: 'Structural basis for the activity of the RSK-specific inhibitor, SL0101' BIOORG MED CHEM vol. 15, no. 14, 2007, pages 5018 - 5034, XP022103888
- CABRERA, M. ET AL.: 'Synthetic chalcones, flavanones, and flavones as antitumoral agents: biological evaluation and structure-activity relationships' BIOORG MED CHEM vol. 15, no. 10, 2007, pages 3356 - 3367, XP022024192

## Description

### TECHNICAL FIELD

The present invention relates to a composition for use in ameliorating cognitive impairment comprising as an active ingredient thereof nobilet in or an analog thereof, and royal jelly.

### BACKGROUND ART

In Japan where the size of the elderly population is continuing to grow, the prevention and treatment of cognitive impairment attributable to aging, including impairment of the ability to concentrate, memory recall, organizational ability, planning ability and problem-solving ability, and particularly the representative disease thereof in the form of Alzheimer's disease, is becoming an important issue.

Nobiletin is a polymethoxyflavonoid contained in numerous citrus fruits that has been reported to have an information transmission promoting action that manages memory as well as inhibits facillitation of learning disabilities, and is known to be useful as an ameliorant for Alzheimer's disease and other forms of cognitive impairment (Patent Documents 1 and 2).

Royal jelly is a substance secreted from the pharyngeal glands of young worker honeybees, and is consumed as nourishment by larvae that become queen bees as well as adult queen bees. This substance is the only energy source for queen bees during their entire life span, which is forty times longer than that of worker bees. In addition, royal jelly contains incomparably higher levels of vitamins, minerals and amino acids than ordinary honey, and is also known to be rich in protein and contain various nutrients. However, the effects of royal jelly on cognitive impairment are still unknown.

Patent Document 1: Japanese Unexamined Patent Publication No. 2002-60340
Patent Document 2: International Patent Publication No. WO2005-082351

JP2002-060340 aims at obtaining a new neurite elongation agent showing neurite elongation effect on neurocytes without adverse drug reactions. It was found that a polyalkoxyflavonoid having a specific structure, particularly tangeritin or nobiletin exhibits neurite elongation effect. Further, it was found out that an extract from a plant belonging to the family Rutaceae containing this polyalkoxyflavonoid also exhibits the neurite elongation effect.

A newspaper article in "[Ryoyaku Kuchi ni Umashi] Rojinsei Chihosho Seiriteki Roka to Byoteki Roka" SANKEI SHIMBUN, 15 February 1999, page 17, XP008143126 TOKYO CHOKAN contains a description that royal is good for the prevention of Alzheimer's disease.

### DISCLOSURE OF THE INVENTION

### Problems to be Solved by the Invention

An object of the present invention is to provide a cognitive impairment ameliorant useful for the prevention or treatment of cognitive impairment, and particularly preferable for Alzheimer's disease.

### Means for Solving the Problems

As a result of conducting extensive studies, the inventors of the present invention found for the first time that a composition comprising nobiletin or an analog thereof, and royal jelly, demonstrates extremely superior effects in a learning disability model, thereby leading to completion of the present invention.

### BRIEF DESCRIPTION OF THE DRAWINGS

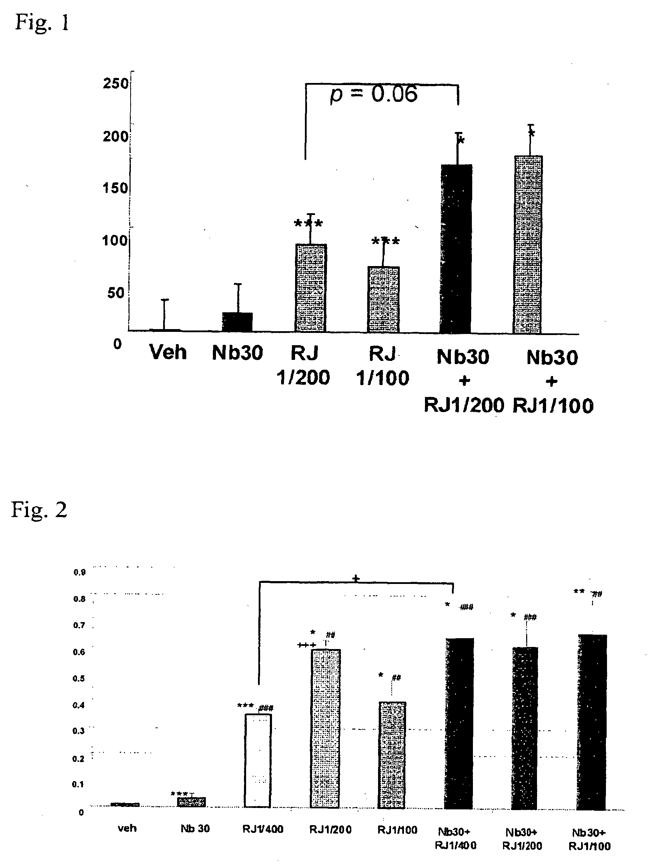

FIG. 1 indicates the effects of royal jelly and nobiletin on ERK phosphorylation in PC12D cells. Data is shown as the mean ± standard error (n=3). Asterisks (*) indicate a level of significance of p<0.05 relative to a vehicle, while triple asterisks (***) indicate a level of significance of p<0.001 relative to a vehicle (n=3). VeH of the horizontal axis indicates the vehicle, Nb30 indicates 30 µM nobiletin, RJ1/200 and RJ1/100 indicate 200-fold and 100-fold dilutions of royal jelly, respectively, and RJ1/200+Nb30 and RJ1/100+Nb30 indicate 200-fold and 100-fold dilutions of royal jelly and 30 µM nobiletin, respectively. The relative value of phosphorylated ERK to total ERK is plotted on the vertical axis.
FIG. 2 indicates the enhancing effects of nobiletin on royal jelly-induced stimulation of ERK phosphorylation in PC12D cells. The graphs show typical results obtained from at least three independent rounds of testing (upper portion). Values on the vertical axis represent measured concentration values relative to ERK phosphorylation in PC 12D cells, and indicate relative values versus values of a control group (0.1% DMSO). Asterisks (*) indicate a level of significance of p<0.05, double asterisks indicate a level of significance ofp<0.01, and triple asterisks (***) indicate a level of significance of p<0.005 relative to a vehicle. Double sharp marks (##) indicate a level of significance of p<0.01 while triple sharp marks (###) indicate a level of significance ofp<0.005 relative to 30 µM nobiletin. Plus marks (+) indicate a level of significance of p<0.05 and triple plus marks (+++) indicate a level of significance of p<0.01 relative to 400-fold diluted royal jelly. Values are shown as the mean ± standard error (n=3). Veh indicates a vehicle, Nb30 indicates 30 µM nobiletin, RJ1/400, RJ1/200 and RJ1/100 indicate 400-fold, 200-fold and 100-fold dilutions of royal jelly, respectively, and RJ1/400+Nb30, RJ1/200+Nb30 and RJ1/100+TNb30 indicate 400-fold, 200-fold and 100-fold dilutions of royal jelly and 30 µM nobiletin, respectively.

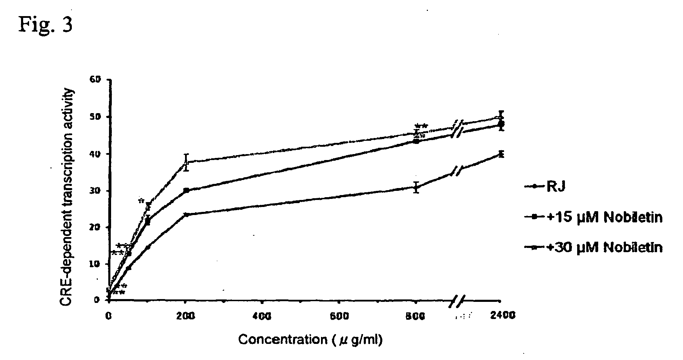

FIG. 3 indicates the effects of combined use of royal jelly (RJ) and nobiletin on CRE-dependent transcription in PC12D cells. Data is shown as the mean ± S.E.M. (n=4). Asterisks (*) indicate a level of significance of p<0.05 relative to RJ, while double asterisks (**) indicate a level of significance of p<0.001 relative to RJ.

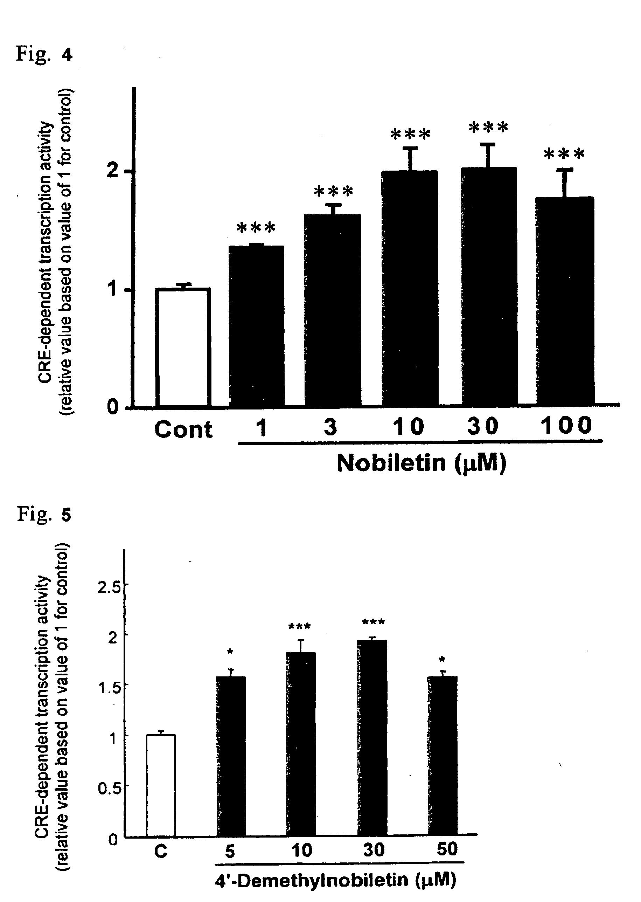

FIG. 4 indicates concentration-dependent effects of nobiletin on CRE-dependent transcription in cultured rat hippocampal neuronal cells. Cells were inoculated at a density of 8 x 10⁴ cells/48 well plate followed by holding the plate for 10 to 14 days. Next, the cells were transfected for 16 hours using a luciferase reporter construct demonstrating CRE-dependent transcription. Following transfection, the cells were treated for 8 hours with different concentrations of 4-demethylated nobiletin. Data is shown as the mean ± S.E.M. (n=6). Asterisks (*) indicate a level of significance of p<0.05 relative to a control, while double asterisks (**) indicate a level of significance of p<0.001 relative to a control.
FIG. 5 indicates concentration-dependent effects of 4-demethylated nobiletin on CRE-dependent transcription in cultured rat hippocampal neuronal cells. Cells were inoculated at a density of 8 x 10⁴ cells/48 well plate followed by holding the plate for 10 to 14 days. Next, the cells were transfected for 16 hours using a luciferase reporter construct demonstrating CRE-dependent transcription. Following transfection, the cells were treated for 8 hours with different concentrations of 4-demethylated nobiletin. Data is shown as the mean ± S.E.M. (n=6). Asterisks (*) indicate a level of significance of p<0.05 relative to a control, while double asterisks (**) indicate a level of significance of p<0.001 relative to a control.

### BEST MODE FOR CARRYING OUT THE INVENTION

The nobiletin used in the present invention comprises 5,6,7,8,3',4'-hexamethoxyflavone, a pharmaceutically acceptable salt thereof, or a hydrate or solvate thereof.

The nobiletin of the present invention can be chemically synthesized using methods known among persons with ordinary skill in the art. In addition, it can also be extracted from plants of the citrus family. This includes, for example, the nobiletin analog, 4'-hydroxy-5,6,7,8,3'-pentamethoxyflavone (4-demethylated nobiletin), pharmacologically acceptable salts thereof, as well as hydrates or solvates thereof.

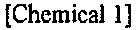

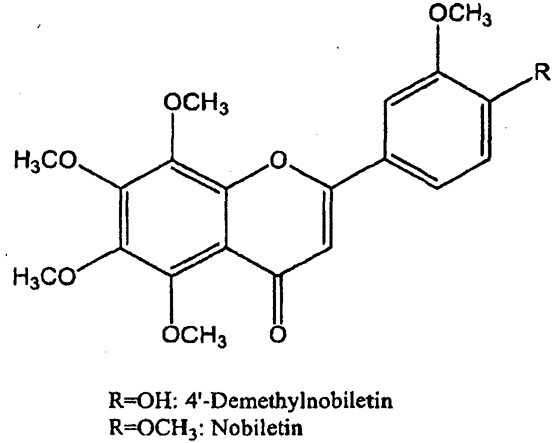

Examples of plants of the citrus family include Citrus depressa, Citrus unshiu, Citrus tangerina, Citrus erythrosa, Citrus aurantium, Citrus deliciosa, Citrus grandis, Citrus kinokuni, Citrus reticulata, Citrus tachibana and Citrus sunki.

The extract of a citrus family plant may be extracted from a plant in the natural state or dried state after harvesting. Examples of extracted sites include the fruit, skin, seeds, leaves, branches, roots or flowers of mature or immature. Extracts are preferable obtained from the fruit or skin.

There are no particular limitations on royal jelly able to be used in the present invention, and commercially available royal jelly can be used. The royal jelly is preferably a product manufactured by Japan Royal Jelly Co., Ltd. In the present invention, the royal jelly is royal jelly freeze-dried powder (FD powder) unless specifically indicated otherwise.

In the present invention, the content of nobiletin is 0.0025 to 2.00% by weight, preferably 0.005 to 1.50% by weight, and more preferably 0.01 to 1.00% by weight. In addition, the content of royal jelly as royal jelly FD powder is 3 to 60% by weight, preferably 5 to 45% by weight and more preferably 10 to 30% by weight.

In the present invention, the ratio of the content of nobiletin to the content of royal jelly is 2:3 to 1:24000, preferably 3:10 to 1:9000 and more preferably 1:10 to 1:600.

The composition for use in ameliorating cognitive impairment of the present invention can be incorporated in a prescription drug, quasi drug or food and the like. In addition, a dendrite elongation agent of the present invention can be used by oral or parenteral administration.

Examples of forms of prescription drugs include tablets, capsules, granules or syrup. These prescription drugs can be produced by using additives normally used in the production of prescription drugs.

Although there are no particular limitations on the dosage of the composition for use in ameliorating cognitive impairment of the present invention, in the case of oral administration, for example, the dosage is 10 to 60 mg/kg of body weight per day and preferably 20 mg/kg of body weight per day, while in the case of parenteral administration, the dosage if 2 to 6 mg/kg of body weight per day and preferably 2 to 3 mg/kg of body weight per day. The aforementioned dosages can be administered once per day or divided into 2 to 3 administrations per day, and the dosage can be suitably adjusted according to age, disease status and symptoms.

Specific examples of additives include lactose, dextrin, sucrose, mannitol, cornstarch, sorbitol, microcrystalline cellulose and polyvinyl pyrrolidone, and these additives can be used alone or in a suitable combination thereof. These prescription drugs can be produced using methods suitable for each drug form in accordance with that described in the Japanese Pharmacopeia. In addition, flavoring agents, colorants, sweeteners and the like can also be suitably used. The contents of these additives can be suitably selected by a person with ordinary skill in the art.

Examples of forms of quasi drugs include tablets, capsules, granules, jelly and drinks. These quasi drugs can be produced using additives normally used in the production of quasi drugs. Moreover, these quasi drugs can also contain other active ingredients such as vitamins. In addition, additives such as sweeteners, flavoring agents, colorants or antioxidants can also be used alone or in a suitable combination thereof. These quasi drugs can be produced by methods commonly known among persons with ordinary skill in the art.

Examples of the forms of foods include noodles, pasta, powders, tablets, jelly and liquids (beverages). These foods can be produced by suitably using various food materials. Specific examples of food materials include rice, wheat, corn, potatoes, sweet potatoes, soybeans, powdered seaweed, candy, lactose, glucose, fructose, sucrose and mannitol, and these can be used alone or in a suitable combination thereof. A desired form can be obtained by using water and the like as necessary. Moreover, additives such as flavoring agents, colorants, sweeteners, edible oils or vitamins can also be suitably added.

Although the following provides a detailed explanation of the present invention through examples thereof, these examples are only intended to be representative of the present invention, and the present invention is not limited thereto.

### EXAMPLES

The cAMP/PKA/ERK/CREB-dependent signaling pathway has been demonstrated to be intimately involved in memory and learning ability of the brain. In recent years, nerve degeneration and neurological deficit attributable to accumulation of amyloid β protein (Aβ) in the brain, along with synaptic plasticity disorders accompanied by nerve cell death due to solubilizing Aβ, have come to be understood to be one of the factors of memory disorders associated with Alzheimer's disease (AD). For example, the long-term potentiation (LTP) of neural transmissions at hippocampal glutamate synapses, which is considered to be one of the cellular mechanisms of learning and memory formation, is a typical example of synapse plasticity. Aβ inhibits PKA/CREB signaling and is known to suppress the occurrence of this LTP. In other words, a food that contains a physiologically active substance capable of improving or suppressing inhibition of signaling by Aβ in this manner is considered to be useful for the improvement and/or prevention of AD.

Therefore, the effects of royal jelly and the effects of the combined used of royal jelly and nobiletin on ERK activity in PC12D cells, which are considered to be model cells of nerve cells, were examined by western blotting using as an indicator thereof the effects of water-soluble components of "Royal Jelly FD Powder" on ERK phosphorylation.

### 1. Experimental Method

### Culturing of PC12D Cells

PC12D cells (Aichi Prefectural County Research Institute) were sub-cultured using Dulbecco's Modified Eagle Medium (DMEM) comprising heat inactivated (56°C, 30 minutes) 5% horse serum (HS: Gibco), 10% fetal calf serum (FCS: CELLect), penicillin (50 units/mL) and streptomycin (50 µg/mL). The cells were cultured in an incubator maintained at 37°C and comprising a mixture of 95% air and 5% CO₂. Furthermore, in this experiment, medium prepared so as to contain 2% HS and 1% FCS was used to dilute the drug.

### Preparation of "Royal Jelly FD Powder" Stock Solution (25%(w/v))

0.25 g of "Royal Jelly FD Powder" (Japan Royal Jelly Co., Ltd.) were weighed out into a sterilized 2 ml tube using a sterilized spatula. Subsequently, sterile PBS(-) was added to bring to a volume of 1 ml inside a laminar flow cabinet. After agitating, the solution was transferred to a refrigerator and allowed to stand undisturbed in a refrigerator overnight while repeating agitation every hour. After agitating on the following day, the solution was centrifuged for 10 minutes at 12000 x g and 4°C. The supernatant was then sterilized by filtration and then stored frozen at -20°C after dividing into individual aliquots.

### Western Blotting

PC12D cells were inoculated into a 35 mm dish at 1 x 10⁶ cells/dish, and after culturing for 24 hours in a CO₂ incubator, the cells were treated with drug. After lysing the drug-treated cells with 90 µl of cell lysis solution (1 mM EDTA, 1% SDS, 10 mM NaF, 10 nM calyculin, 320 nM okadaic acid, 1 mM sodium orthovanadate, 1 mM p-APMSF, 10 µg/ml pepstatin, 10 µg/ml antipain, 10 µg/ml leupeptin, 10 µg/ml chymostatin, 10 µg/ml phorphoramidon and 10 mM HEPES (pH 7.5)), the lysate was recovered in a 1.5 ml tube and heated for 5 minutes at 95°C. Moreover, after subjecting to ultrasonic treatment for 5 minutes in ice water, the lysate was centrifuged for 20 minutes at 14,000 rpm followed by recovery of the supernatant and quantification of the amount of protein. Subsequently, SDS-PAGE sample buffer was added. Following SDS-PAGE, the protein was transferred to a PVDF membrane by western blotting followed by blocking for 2 hours using a blocking buffer (10 mM Tris-HCl, 100 mM NaCl, 0.05% Tween 20 and 5% skim milk: pH 7.4). Subsequently, the membrane was washed with TBST and incubated overnight at 4°C with primary antibody diluted with blocking buffer. The membrane was further washed with TBST and incubated for 2 hours at room temperature with HRP-labeled IgG antibody diluted with blocking buffer followed by again washing with TBST. Bands were detected using the ECL method. Reprobing consisted of removing the antibody using stripping buffer (62.5 mM Tris-HCl, 2% SDS, 100 mM β-mercaptoethanol: pH 7.4) followed by detecting internal standard protein.

### Data Analysis

Experiment values were shown as the mean ± standard error. Student's t-test, analysis of variance and Dunnett's test were used for statistical processing, and a significant difference was judged to be present in the case of a level of significance of less than 5%.

### 2. Results

### Effects of Royal Jelly and Nobiletin on ERK Phosphorylation in PC12D Cells

PC12D cells were inoculated into a 3.5 cm dish at 1 x 10⁶ cells/dish and cultured overnight followed by treating for 15 minutes with low-serum medium comprising various drugs, and lysing the cells with cell lysis solution to obtain a cell extract. The expression levels of p-ERK and total ERK in the cell extract were then analyzed. Furthermore, the royal jelly solutions were used in the experiment after preparing a 25% solution and diluting with low-serum medium.

As a result, royal jelly remarkably promoted phosphorylation of ERK, which is an important constituent molecule of the cAMP/PKA/ERK/CREB-dependent signaling pathway that is known to play an important role in long-term potentiation of synaptic transmission, one of the cellular mechanisms of memory and learning, in PC12D cells at concentrations corresponding to dilution factors of 400-fold to 100-fold (0.0625 to 0.25% (w/v)) (FIGS. 1 and 2). On the other hand, weak promoting action of ERK phosphorylation was observed in a 30 µM nobiletin treatment group (FIG. 2).

### Effects of Nobiletin on Promotion of Royal Jelly-Induced ERK Phosphorylation in PC 12D Cells

The following extremely interesting results were also obtained. Namely, 30 µM nobiletin, which only demonstrated weak promoting action of ERK phosphorylation when used alone, was determined to further enhance the remarkable ERK phosphorylation promoting activity of royal jelly. This finding strongly indicates the possibility that the combined use of royal jelly and nobiletin prominently promotes cAMP/PKA/ERK/CREB-dependent signaling intimately involved with memory and learning (FIG. 2).

A study was conducted of the action of royal jelly alone or the combined use of royal jelly and nobiletin on cAMP/PKA/ERK/CREB-dependent signaling in PC12D cells. Culturing of PC12D cells and preparation of "Royal Jelly FD Powder" stock solution (25% (w/v)) were carried out in the same manner as previously described.

### 1. Experimental Method

### Sub-culturing of Rat Hippocampal Neuronal Cells

A pregnant SD rat (E18) was sacrificed by cervical dislocation under ether anesthesia. After subsequently disinfecting the abdomen with 70% EtOH, the fetus was excised by making a midline incision in the abdomen. Moreover, the brain was excised from the rat fetus and the hippocampus was isolated using a stereo microscope. Culturing of hippocampal neuronal cells was carried out using the Sumitomo Nerve Cell Culture System manufactured by Sumitomo Bakelite Co., Ltd. The hippocampal tissue was dispersed with a dispersion solution comprising papain enzyme (Sumilon) and the supernatant was removed after centrifuging for 4 minutes at 1,000 rpm. The resulting pellet was dispersed with a dispersion solution (Sumilon), an isolation solution (Sumilon) was added to the cells after being adequately dispersed by pipetting, and the supernatant was removed after centrifuging for 5 minutes at 800 rpm. The resulting pellet was suspended using Neurobasal medium (comprising 500 ml of phenol red-free Neurobasal medium, 10 ml of 50x B-27 supplement, 0.5 mM L-glutamine and 0.005% penicillin-streptomycin), the suspension was inoculated into a polylysine-coated 48-well plate at 8 x 10⁴ cells/well, and after culturing for 1 day, the cells were washed with PBS following by replacing with fresh medium and culturing with medium comprising 10 µM AraC by replacing half of the medium every 2 to 3 days.

### Transfection and Reporter Gene Assay

PC12D cells were inoculated into a 48-well plate at 8 x 10⁴ cells/well, and after culturing for 24 hours in growth medium, co-transfection was carried out using Lipofectamine® 2000 reagent (Invitrogen) at a concentration of pCRE reporter gene plasmid (firefly luciferase) of 0.1 µg/well and at a concentration of internal standard pRG-TK plasmid (Renilla mushroom luciferase) of 0.01 µg/well 16 hours after transfection, the medium was replaced with medium comprising various drugs. After treating for 5 hours with medium comprising various concentrations of drugs, the medium was aspirated followed by lysing and recovering the cells by addition of passive lysis buffer (Dual-Luciferase Reporter Assay System, Cat. No. E1960 (Promega)). Furthermore, activities of the firefly and Renilla mushroom luciferases were measured with a luminometer (Mini Lumat LB9506 (Berthold)) using the Dual-Luciferase Reporter Assay System (Promega).

On the other hand, hippocampal neuronal cells prepared according to the method described above were cultured for 10 to 14 days with Neurobasal medium, transfected with reporter plasmid (0.1 µg/well) and Renilla mushroom pRG-TK plasmid (0.01 µg/well) by lipofection, and treated with drug for a fixed period of time. Measurement of transcription activity was carried out using the Dual-Luciferase® Reporter Assay System manufactured by Promega.

### Statistical Analysis

Results obtained from the reporter gene assay were evaluated using one-way ANOVA (Tukey). The presence of a significant difference was tested based on a level of significance of 5% on both sides, and a significant difference was judged to be present in the case of a level of significance of less than 5%.

A study was made of the effects of combined use of royal jelly (RJ) and nobiletin on CRE-dependent transcription in PC12D cells.

As shown in FIG. 3, although royal jelly alone demonstrated an effect on CRE-dependent transcription in PC12D cells, that effect was enhanced by combined use of 15 µM or 30 µM nobiletin, and the test group in which the concentration of nobiletin was increased to 30 µM at the time of combined use was determined to enhance effects on CRE-dependent transcription more than that at a concentration 15 µM.

Moreover, a study was also conducted on the effects of nobiletin on CRE-dependent transcription in sub-cultured rat hippocampal neuronal cells.

As a result of examining the effects of nobiletin on CRE-dependent transcription using the reporter gene assay method, nobiletin was determined to concentration-dependently promote CRE-dependent transcription in hippocampal neuronal cells.

As shown in FIG. 4, as a result of examining the effects of nobiletin on CRE-dependent transcription using the reporter gene assay method, nobiletin was determined to concentration-dependently promote CRE-dependent transcription in hippocampal neuronal cells. In addition, as shown in FIG. 6, 4-demethylated nobiletin was also determined to concentration-dependently promote CRE-dependent transcription in the same manner as nobiletin.

The following provides an example of a method for producing 4-demethylated nobiletin. However, the method used to produce 4-demethylated nobiletin is not limited to the method described here.

A scheme for organic synthesis of 4-demethylated nobiletin is shown below.

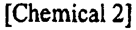

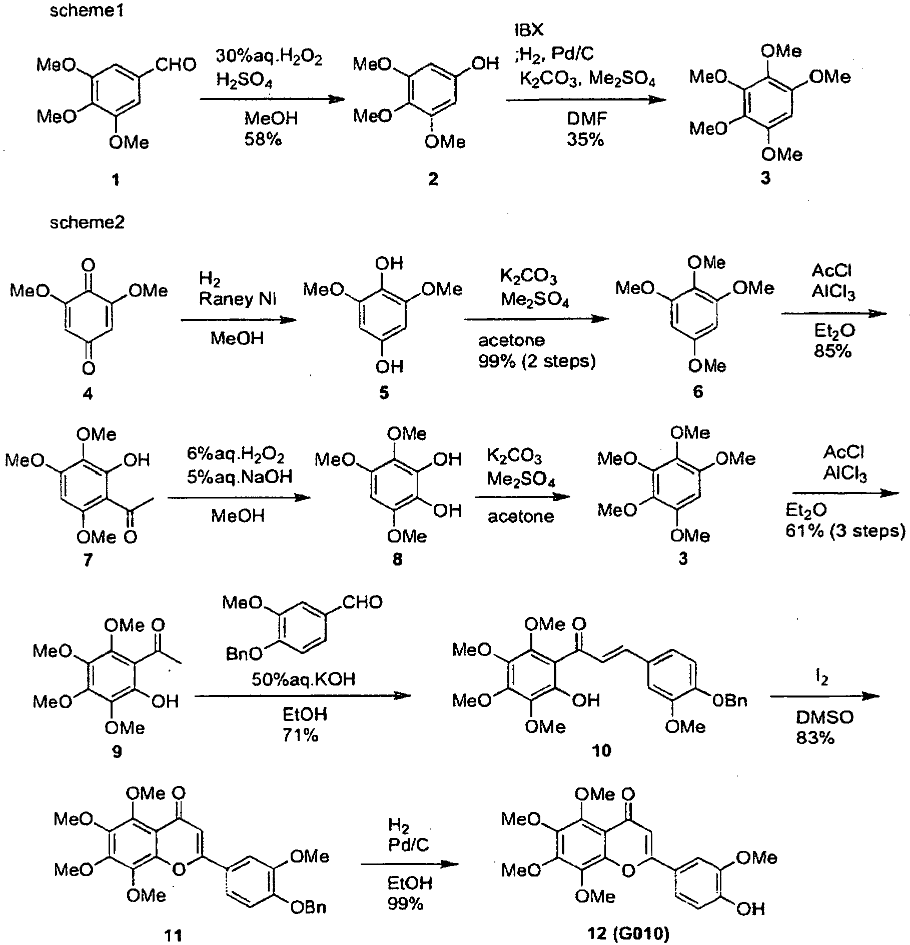

### 3,4,5-Trimethoxyphenol (2)

After adding concentrated sulfuric acid (0.54 ml, 10.2 mmol) to an MeOH solution (20.4 ml, 0.5 M) of 3,4,5-trimethoxybenzaldehyde (2.0 g, 10.2 mmol) while cooling with ice, a 30% aqueous hydrogen peroxide solution (3.5 ml, 30.6 mmol) was added slowly. After stirring for 15 minutes at room temperature, a 10% aqueous sodium hydroxide solution and sodium sulfite were added while cooling with ice to stop the reaction. After washing an organic layer extracted with AcOEt with brine, the organic layer was dried with MgSO₄ followed by distilling off the solvent under reduced pressure. The residue was subjected to a silica gel chromatography, and 3,4,5-trimethoxyphenol (1.08 g, 7.0 mmol, 58%) was obtained in the form of a colorless solid from a elution part of AcOEt/hexane (5:6 v/v).

Colorless plate (AcOEt/hexane): mp 145-147 °C. IR (CHCl₃) : 3303, 1612, 1129 cm⁻¹. ¹H-NMR (400MHz, CDCl₃) δ : 6.09 (2H, s), 5.90 (1H, s), 3.79 (3H, s), 3.76 (6H, s). ¹³C-NMR (100 MHz, CDCl₃) δ : 153.6, 152.7, 131.4, 93.0, 61.0, 55.9. MS m/z : 184 (M⁺). HRMS Calcd. for C₉H₁₂O₄ : 184.0736. Found : 184.0715.

### 1,2,3,4,5-Pentamethoxybenzene (3)

2-iodoxybenzoic acid (IBX) (160 mg, 5.7 mmol) were added to a DMF solution (3.6 ml, 0.15 M) of 3,4,5-trimethoxyphenol (0.10 g, 0.54 mmol) at room temperature followed by stirring for 30 minutes. After adding 1% Pd/C (10 mg), the system was replaced with a hydrogen atmosphere followed by stirring for 1 day. After returning the system to an argon atmosphere, potassium carbonate (220 mg, 1.6 mmol) and dimethyl sulfate (0.13 ml, 1.4 mmol) were added, and after additionally stirring for 3 hours at room temperature, the reaction solution was filtered with Celite and the solvent was distilled off under reduced pressure. After washing the organic layer, obtained by extracting the residue with Et₂O, with brine, the organic layer was dried with MgSO₄ and the solvent was distilled off under reduced pressure. The residue was subjected to a silica gel chromatography, and 1,2,3,4,5-pentamethoxybenzene (42 mg, 0.18 mmol, 34%) was obtained in the form of a colorless solid from a elution part of AcOEt/hexane (5:7 v/v).

Colorless needle (hexane) : mp 59-61°C. IR (CHCl₃) : 1108 cm⁻¹. ¹H-NMR (400 MHz, CDCl₃) δ : 6.30 (1H, s), 3.95 (3H, s), 3.85 (6H, s), 3.83 (6H, s). ¹³C-NMR (100 MHz, CDCl₃) δ : 149.1, 147.9, 136.7, 93.7, 61.4, 61.3, 56.5. MS m/z : 228 (M⁺). HRMS Calcd. for C₁₁H₁₆O₅ : 228.0998. Found : 228.0983. Anal. Calcd. for C₁₁H₁₆O₅ : C, 57.88. H, 7.07. Found : C, 57.77. H, 7.00.

### 1,2,3,5-Tetramethoxybenzene (6)

2,6-dimethoxy[1,4]benzoquinone (10 g, 58.8 mmol) was added to an MeOH solution (100 ml, 0.59 M) of Raney nickel (3.5 g) followed by stirring for 6 hours at room temperature in a hydrogen atmosphere. The reaction solution was filtered with Celite and the solvent was distilled off under reduced pressure to obtain crude 2,6-dimethoxybenzene-1,4-diol.

Potassium carbonate (41 g, 297 mmol) and dimethyl sulfate (14 ml, 147 mmol) were added to an acetone solution (100 ml, <0.59 M) of crude 2,6-dimethoxybenzene-1,4-diol (<58.8 mmol) followed by additionally stirring for 3 hours while heating at reflux. After washing an organic layer extracted with AcOEt with brine, the organic layer was dried with MgSO₄ followed by distilling off the solvent under reduced pressure. The residue was subjected to a silica gel chromatography, and 1,2,3,5-tetramethoxybenzene (11.5 g, 158.1 mmol, 99%) was obtained in the form of a colorless oil from a elution part of AcOEt/hexane (1:2 v/v).

Colorless oil IR (CHCl₃) : 2931, 1594, 1506, 1129 cm⁻¹. ¹H-NMR (400 MHz, CDC13) δ : 6.15 (2H, s), 3.85 (6H, s), 3.79 (3H, s), 3.79 (3H, s). ¹³C-NMR (100 MHz, CDCl₃) δ : 156.2, 153.7, 132.4, 91.7, 61.0, 56.1, 55.5. MS m/z : 198 (M⁺). HRMS Calcd. for C₁₀H₁₄O₄ : 198.0892. Found : 198.0885.

### 2'-Hydroxy-3',4',6'-trimethoxyacetophenone (7)

AlCl₃ (26 g, 192 mmol) was added to an Et₂O solution (200 ml, 0.29 M) of 1,2,3,5-tetramethoxybenzene (11.5 g, 58.1 mmol) while cooling with ice. AcCl (5.4 ml, 75.5 mmol) was then added slowly followed by stirring for 2 hours at room temperature. The reaction was stopped by adding water until the aluminum dissolved while cooling with ice. After washing an organic layer extracted with Et₂O with brine, the organic layer was dried with MgSO₄ followed by distilling off the solvent under reduced pressure. The residue was subjected to a silica gel chromatography, and 2'-hydroxy-3',4',6'-trimethoxyacetophenone (11.1 g, 49.1 mmol, 85%) was obtained in the form of a yellow solid from a elution part of AcOEt/hexane (1:1 v/v).

Yellow needle (AcOEt) : mp 114-116 °C. IR (CHCl₃) : 1626, 1587, 1126 cm⁻¹. ¹H-NMR (400 MHz, CDCl₃) δ : 13.80 (1H, s), 5.97 (1H, s), 3.94 (3H, s), 3.90 (3H, s), 3.82 (3H, s), 2.62 (3H, s). ¹³C-NMR (100 MHz, CDCl₃) δ : 203.7, 1.59.0, 158.8, 158.4, 130.5, 106.3, 86.4, 60.7, 55.9, 55.5, 33.2. MS m/z : 226 (M⁺). HRMS Calcd. for C₁₁H₁₄O₅ : 226.0841. Found : 226.0829. Anal. Calcd. for C₁₁H₁₄O₅ : C, 58.40. H, 6.24. Found : C, 58.37. H, 6.18.

### 2'-Hydroxy-3',4',5',6'-tetramethoxyacetophenone (9)

An aqueous sodium hydroxide solution (40 ml, 48 mmol) was added to an MeOH solution (130 ml, 0.37 M) of 2-hydroxy-3',4',6'-trimethoxyacetophenone (1.08 g, 47.7 mmol) while cooling with ice followed by slowly adding a 6% aqueous hydrogen peroxide solution (82 ml, 143 mmol). After stirring for 30 minutes at room temperature, 10% hydrochloric acid and sodium sulfite were added while cooling with ice to stop the reaction. After washing an organic layer extracted with AcOEt with brine, the organic layer was dried with MgSO₄ followed by distilling off the solvent under reduced pressure to obtain crude 3,4,6-trimethoxybenzene-1,2-diol in the form of a colorless solid.

Potassium carbonate (170 g, 1.2 mol) and dimethyl sulfate (22 ml, 239 mmol) were added to an acetone solution (150 ml) of crude 3,4,6-trimethoxybenzene-1,2-diol followed by heating at reflux for 3 hours. After washing an organic layer extracted with AcOEt with brine, the organic layer was dried with MgSO₄ followed by distilling off the solvent under reduced pressure. The residue was subjected to a silica gel chromatography, and 1,2,3,4,5-pentamethoxybenzene (11.5 g, 158.1 mmol, 99%) was obtained in the form of inseparable mixture with dimethyl sulfate from a elution part of AcOEt/hexane (5:7 v/v).

AlCl₃ (42 g, 313 mmol) was added to an Et₂O solution (330 ml) of crude 1,2,3,4,5-pentamethoxybenzene while cooling with ice. Subsequently, AcCl (14 ml, 187.5 mmol) was added slowly followed by stirring for 2 hours at room temperature. The reaction was stopped by adding water until the aluminum dissolved while cooling with ice. After washing an organic layer extracted with Et₂O with brine, the organic layer was dried with MgSO₄ followed by distilling off the solvent under reduced pressure. The residue was subjected to a silica gel chromatography, and 2'-hydroxy-3',4',5',6'-tetramethoxyacetophenone (4.5 g, 17.6 mmol, 37%) was obtained in the form of a yellow oil from a elution part of AcOEt/hexane (1:3 v/v).

Yellow oil. IR (CHCl₃) : 2985, 1621, 1409, 1064 cm⁻¹. ¹H-NMR (400 MHz, CDCl₃) δ : 13.12 (1H, s), 4.08 (3H, s), 3.96 (3H, s), 3.86 (3H, s), 3.81 (3H, s), 2.68 (3H, s). ¹³C-NMR (100 MHz, CDCl₃) δ : 204.2, 154.6, 153.7, 151.4, 138.0, 136.8, 110.4, 61.3, 61.2, 61.1, 61.0, 32.2. MS m/z : 256 (M⁺). HRMS Calcd. for C₁₂H₁₆O₆ : 256.0947. Found : 256.0963.

### 3-(4-Benzyloxy-3-methoxyphenyl)-1-(2-hydroxy-3,4,5,6-tetramethoxyphenyl)pro penone (10)

A 50% potassium hydroxide solution (0.37 ml, 3.3 mol) was dropped in an EtOH solution (5.0 ml, 0.19 M) of 4-benzyloxy-3-methoxybenzaldehyde (340 mg, 1.41 mmol) and 2'-hydroxy-3',4',5',6'-tetramethoxyacetophenone (240 mg, 0.94 mmol) at room temperature. After stirring for 24 hours, the reaction was stopped by adding 10% hydrochloric acid while cooling with ice. After washing the organic layer with brine, the organic layer was dried with MgSO₄ and the solvent was distilled off under reduced pressure. The residue was subjected to a silica gel chromatography, and 3-(4-benzyloxy-3-methoxyphenyl)-1-(2-hydroxy-3,4,5,6-tetramethoxyphenyl)-propenone (0.32 mg, 0.66 mmol, 71%) was obtained in the form of a yellow solid from a elution part of AcOEt/hexane (1:2 v/v).

Yellow needle (AcOEt/hexane) : mp 99-100 °C. IR (CHCl₃) : 1626, 1509, 1408, 1259, 1055 cm⁻¹. ¹H-NMR (400 MHz, CDCl3) δ: 13.26 (1H, s), 7.82 (1H, d, J = 15.6 Hz), 7.78 (1H, d, J = 15.6 Hz), 7.45-7.31 (5H, m), 7.17 (1H, d, J = 8.4 Hz), 7.16 (1H, s), 6.90 (1H, d, J = 8.4 Hz), 5.21 (2H, s), 4.09 (3H, s), 3.95 (3H, s), 3.89 (3H, s), 3.87 (3H, s), 3.86 (3H, s). ¹³C-NMR (100 MHz, CDCl₃) δ : 193.3, 154.8, 153.2, 150.7, 150.4, 149.6, 144.0, 138.3, 137.1, 136.4, 128.5, 128.4, 127.9, 127.1, 124.2, 122.8, 113.4, 111.0, 110.9, 70.8, 62.2, 61.6, 61.3, 61.0, 56.0. Anal. Calcd. for C₂₂H₂₈O₈ : C, 67.49. H, 5.87. Found : C, 67.28. H, 5.83.

### 2-(4-benzyloxy-3-methoxyphenyl)-5,6,7,8-tetramethoxychromen-4₋one (11)

Iodine (3 mg, 0.01 mmol) was added to a DMSO solution (1.5 ml, 0.13 M) of 3-(4-benzyloxy-3-methoxyphenyl)-1-(2-hydroxy-3,4,5,6-tetramethoxyphenyl)-propenone (95 mg, 0.20 mmol) at room temperature followed by stirring for 1.5 hours at 150°C. The reaction was then stopped by adding sodium sulfite while cooling with ice. After washing an organic layer extracted with Et₂O with brine, the organic layer was dried with MgSO₄ followed by distilling off the solvent under reduced pressure. The residue was subjected to a silica gel chromatography, and 2-(4-benzyloxy-3-methoxyphenyl)-5,6,7,8-tetramethoxychromen-4-one (79 mg, 0.16 mmol, 83%) was obtained in the form of a yellow solid from a elution part of AcOEt/hexane(3:1 v/v).

Slightly yellow plate (AcOEt/hexane): mp 109-111 °C. IR (CHCl₃) : 1641, 1514, 1361 cm⁻¹. ¹H-NMR (400 MHz, CDCl₃) δ : 7.49-7.32 (7H, m), 6.99 (1H, d, J = 8.5 Hz), 6.60 (1H s), 5.24 (2H, s), 4.10 (3H, s), 4.01 (3H, s), 3.98 (3H, s), 3.95 (6H, s). ¹³C-NMR (100 MHz, CDCl₃) δ : 177.2, 160.8, 151.3, 150.9, 149.7, 148.3, 147.6, 144.0, 137.9, 136.2, 128.6, 128.0, 127.1, 124.2, 119.4, 114.8, 113.4, 108.9, 106.9, 70.9, 62.3, 62.0, 61.8, 61.7, 56.1. Anal. Calcd. for C₂₇H₂₆O₈ : C, 67.77. H, 5.48. Found : C, 67.55. H, 5.58.

### 2-(4-hydroxy-3-methoxyphenyl)-5,6,7,8-tetramethoxychromen-4-one (12, G010)

2-(4-benzyloxy-3-methoxyphenyl)-5,6,7,8-tetramethoxychromen-4-one (79 mg, 0.17 mmol) was added to an EtOH solution (2 ml, 0.08 M) of Pd/C (8 mg) followed by stirring for 2 hours at room temperature in a hydrogen atmosphere. The reaction solution was filtered with Celite and the solvent was distilled off under reduced pressure. The residue was subjected to a silica gel chromatography, and G010 (64 mg, 0.17 mmol, 99%) was obtained in the form of a yellow solid from a elution part of AcOEt

Colorless prism (AcOEt/hexane) : mp 155-156°C. IR (CHCl₃) : 3201, 1634, 1515, 1355, 1076 cm⁻¹. ¹H-NMR (400 MHz, CDCl₃) δ: 7.53 (1H, d, J = 8.3 Hz), 7.40 (1H, s), 7.05 (1H, d, J = 8.3 Hz), 6.60 (1H, s), 6.13 (1H, m), 4.09 (3H, s), 4.04 (3H, s), 3.97 (3H, s), 3.93 (6H, s). ¹³C-NMR (100 MHz, CDCl₃) δ : 177.4, 161.2, 151.4, 149.1, 148.3, 147.6, 147.0, 144.0, 138.0, 123.4, 120.2, 115.1, 114.7, 108.2, 106.5,62.2,61.9, 61.7, 61.6, 56.0. Anal. Calcd. for C₂₀H₂₀O₈ : C, 61.85. H, 5.19. Found : C, 61.72. H, 5.29.

In the synthesis method described above, the oxidative cyclization reaction of 2'-hydroxychalcone to flavone using I₂/DMSO (flavone backbone synthesis) is described in A.M.S. Silva, D.C.G.A. Pinto and J.A.S. Cavaleiro, Tetrahedron Lett., 1994, 35, 5899-5092, the conversion of compound 1 to compound 2 (oxidation of aromatic aldehyde to phenol) is described in M. Matsumoto, H. Kobayashi and Y. Hotta, J. Org. Chem., 1984, 49, 4740, the conversion of compound 2 to compound 3 (synthesis of catechol from phenol) is described in D. Magdziak, A.A. Rodriguez, R.W. Van De Water and T.R.R. Pettus, Org. Lett., 2002, 4, 285-288, the alternative synthesis of compound 3 (scheme 2, synthesis of compound 3 from compound 4) is described in M. Tsukayama, Y. Kawamura, T. Ishizuka, S. Hayashi and F. Torii, Heterocycles, 2003, 60, 2775-2784, and the synthesis of compound 9 (Friedel-Crafts acetylation) is described in M. Tsukayama, E. Kusunoki, M.M. Hossain, Y. Kawamura and S. Hayashi, Heterocycles, 2007, 71, 1589-1600.

## Claims

1. A composition for use in ameliorating cognitive impairment comprising as an active ingredient nobiletin or an analog thereof, and royal jelly

2. A composition for use in ameliorating cognitive impairment according to claim 1, comprising as an active ingredient thereof nobiletin or an analog thereof, royal jelly, as well as Rhodiola sacra extract, Ginkgo biloba leaf extract and ferulic acid.

## Patentansprüche

1. Zusammensetzung zur Verwendung bei der Linderung von kognitiver Beeinträchtigung, umfassend als einen aktiven Inhaltsstoff Nobiletin oder ein Analog hiervon sowie Gelee Royal.

2. Zusammensetzung zur Verwendung bei der Linderung von kognitiver Beeinträchtigung gemäß Anspruch 1, umfassend als einen aktiven Inhaltsstoff hiervon Nobiletin oder ein Analog hiervon, Gelee Royal sowie Rhodiola Sakra-Extrakt, Ginkgo biloba-Blätterextrakt und Ferulasäure.

## Revendications

1. Composition pour son utilisation dans l'amélioration des troubles cognitifs comprenant comme ingrédient actif la nobilétine ou un analogue de celle-ci, et de la gelée royale.

2. Composition pour son utilisation dans l'amélioration des troubles cognitifs selon la revendication 1, comprenant comme ingrédient actif de celle-ci la nobilétine ou un analogue de celle-ci, et de la gelée royale, ainsi que de l'extrait de Rhodia sacra, de l'extrait de feuilles de Ginkgo biloba et de l'acide férulique.
